# EUROPEAN PATENT APPLICATION

(11) **EP 1 136 064 A2**
(43) Date of publication of application: **26.09.2001**
(21) Application number: 01106007.6
(22) Date of filing: 12.03.2001
(51) Int. Cl.: A61K 7/48

(54) **Method for improving the apperance of skin and topical compositions for practicing the same**

(30) Priority: 21.03.2000 US 190988 P; 10.04.2000 US 195907 P; 28.11.2000 US 723508
(71) Applicant: AVON PRODUCTS, INC., New York, NY 10020-1196 (US)
(72) Inventor: Anderson, Glen T., Cortlandt Manor, New York 10566 (US)
(74) Representative: Weitzel, Wolfgang, Dr.-Ing. Patentanwalt

(57) **Abstract**

The present invention relates to novel topical compositions that are useful to enhance the overall appearance of the skin, such as reducing the appearance of fine lines and wrinkles. The present invention, which in brief summary, is a topical cosmetic composition having a spherical and/or substantially spherical optical diffuser particle and crosslinked silicone elastomer. In addition, the present invention includes methods of using such a topical composition and methods for cosmetically improving the appearance of skin.

## Description

### RELATED APPLICATIONS

This application claims priority in U.S. applications Ser. Nos. 60/190,988, filed March 21, 2000 and 60/195,907, filed April 10, 2000.

### BACKGROUND OF THE INVENTION

### 1. Field of the Invention

The present invention relates to methods for cosmetically improving the appearance of skin. More particularly, the present invention relates to novel topical compositions that are useful to enhance the overall appearance of the skin, such as reducing the appearance of fine lines and wrinkles.

### 2. Brief Description of the Prior Art

Due to consumer demand, there are numerous examples of commercial products, which make claims of instant visible benefits. Several of these products use optical diffusers. An optical diffuser is a particle that changes the surface optometrics of skin resulting in a visual blurring and softening of lines and wrinkles. To date, the preferred optical diffusers are soft particles of a wide particle size distribution that have an irregular shape, i.e., not spherical, (hereinafter "irregular optical diffuser particles") and thus a larger surface area. The irregular shape of such irregular optical diffuser particles is presently preferred because it is believed that such irregularities allow the irregular optical diffuser particles to fill in the cracks-and crevices of a wrinkle.

In addition to the irregularity of the shape of each irregular optical diffuser particle, many current cosmetics also utilize a wide distribution range of particle sizes for the following purpose. Larger sized particles will congregate more in the 'valleys' where lines are the deepest, while smaller sized particles will congregate more in the 'peaks' where there are no lines. The objective is a smoother finished skin surface, which reflects light more evenly, and, thus, gives a visible benefit to the consumer.

There is another class of particles commonly used in cosmetics. This second class of particles is formed of hard, spherical particles (hereinafter "spherical particles"), which have a much narrower particle size distribution than the irregular optical diffuser particles. Such spherical particles give a 'ball-bearing' like effect to the feel of a composition, reducing greasiness and improving slip. They are usually non-porous and have low oil absorption characteristics. As compared to the irregular optical diffuser particles noted above, these ingredients have a much more favorable influence on the aesthetics of the cosmetic composition, giving a silky and smooth product. Unfortunately, due to their spherical nature and narrow particle size- distribution, this type of particle does not fit as efficiently in the various sized cracks and crevices of a wrinkle as do the irregular optical diffuser particles. In contrast, spherical particles tend to spread out evenly on the skin, regardless of whether they are in a 'peak' or 'valley'. The end result is that they do not reflect light as evenly and thus are not effective optical diffusers.

However, due to ever-increasing consumer demand to provide aesthetically pleasing cosmetic compositions, which still effectively improve the appearance of skin, there remains a need to provide novel compositions and methods of using such compositions in order to meet the demands of sophisticated consumers.

### SUMMARY OF THE INVENTION

It is an object of the present invention to provide an asthetically pleasing cosmetic composition that improves and enhances the overall appearance of the skin.

It is another object of the present invention to provide a method for improving the appearance of skin.

These objects and others will be provided by the present invention, which in brief summary, is a topical cosmetic composition having spherical and/or substantially spherical particles and crosslinked silicone elastomer in a vehicle. In addition, the present invention includes methods of using such a topical composition.

### DESCRIPTION OF THE INVENTION

A desired cosmetic benefit of skin care products is to provide an overall improvement in the appearance of the skin. It is particularly desirable if such an effect is rendered immediately upon application. Such benefits may include a reduction in the appearance of fine lines and wrinkles, an evening out of skin tone and color, and an overall healthy look.

The disadvantages of spherical particles have been set forth above. However, such spherical particles, if applied to smooth skin with no lines, would in fact reflect the light evenly giving optical benefits similar to the irregularly shaped particles. However, such benefits are not evident when spherical particles are applied to wrinkled skin. It has been discovered that if some other ingredient is present in a topical composition that first smoothes out lines and wrinkles, the spherical particles would then be deposited over this smoother surface so that the light would be reflected more evenly. This would allow the combination of the favorable formulation aesthetics of a spherical particle with the desired optical diffusion effects of the irregular shaped particles.

The present invention provides topical compositions comprising certain compounds that dramatically improve the appearance of the skin. More specifically, these compositions include the use of a crosslinked silicone elastomer in combination with a substantially spherical, preferably perfectly spherical, particle having a narrow particle size distribution.

As used herein, the term "particle size range" refers to the range of diameters from the smallest spherical particle to the largest spherical particle in the material. The term "particle size distribution" refers to the single number that constitutes the difference between the upper and lower limit of the particle size range. The "average particle size" refers to the average diameter of all spherical particles in the material.

Although the actual average particle size of the spherical particles may vary depending upon the needs of the manufacturer with respect to other ingredients and the desired physical properties of the overall composition, it is preferred that the particle size distribution of the spherical particle be within a specific range or value. The particle size range is about 1 micron to about 25 microns. Preferably, the particle size range is about 5 microns to about 20 microns. More preferably, the particle size range is about 8 microns to about 15 microns. Most preferably, the particle size range is about 8 microns to about 10 microns.

The particle size distribution is about 24 microns. Preferably, the particle size distribution is about 15 microns. More preferably, the particle size distribution is about 7 microns. Most preferably, the particle size distribution is about 2 microns. Optimally, the spherical particles are uniform in diameter.

It is preferred that the amount of spherical particles present is about 0.01 wt.% to about 10 wt.%, preferably about 0.5 wt.% to about 5 wt.%, of the total weight of the composition. The most preferred amount of spherical particles present is from 0.5 wt.% to 2.5 wt.% of the total weight of the composition.

Examples of suitable spherical particles include, but are not limited to, silica, nylon, boron nitride, teflon, polyurethane powder, silicone powder, talc, mica, serecite, and mixtures thereof.

The cross-linked silicone elastomer functions by forming a smooth film over the skin, evening out the lines and wrinkles. The spherical particles then deposit over this film, resulting in both an optical diffusion and 'ball-bearing' effect. The ball bearing effect is the result of the applicator's hand rolling on the spherical particles, instead of sliding along the skin as the material is applied. The result is a smoother feel when applied as compared to that of non-uniform particles.

The crosslinked silicone elastomers are formed from a divinyl compound having a siloxane polymer preferably having at least two free vinyl groups. The divinyl compound reacts with Si-H linkages of a polysiloxane backbone. A nonlimiting example of such a polysiloxane backbone is molecularly spherical MQ resin. The crosslinked silicone elastomer can be non-emulsifying (i.e., polyoxyalkylene groups absent) or emulsifying (i.e., polyoxyalkylene groups present). The average molecular weight of the crosslinked silicone elastomer is greater than about 2,000, and preferably greater than about 10,000. More preferably, the average molecular weight of the crosslinked silicone elastomer is greater than about 1 million. Preferably, the average molecular weight of the crosslinked silicone elastomer is less than about 20 million. Most preferably, the average molecular weight of the crosslinked silicone elastomer is from about 1 million to about 20 million. Examples of suitable crosslinked silicone elastomers include, but are not limited to, dimethicone crosspolymer (available from Dow Corning as DC 9040); organopolysiloxane (available from Grant Industries as Gransil GCM); polysilicone-11 (available from Grant Industries as Gransil SR-5CYC); and dimethicone/vinyl dimethicone crosspolymer (available from General Electric as SFE839); and mixtures thereof.

It is preferred that the crosslinked silicone elastomer is present in an amount from about 0.01 wt.% to about 10 wt.%, and preferably from about 0.1 wt.% to about 5 wt.%, of the total weight of the composition. The most preferable amount of cross-linked silicone elastomer is from 0.15 wt.% to about 3 wt.% of the total weight of the composition.

As can be appreciated by those in the art, the ratio of spherical particles to crosslinked silicone elastomer will vary with the molecular weight of the crosslinked silicone elastomer. However, it is preferred that the ratio of spherical particles to crosslinked silicone elastomer is from about 100:1 to about 1:2, more preferably from about 10:1 to about 1:2, and most preferably about 5:1.

The unique aspect of this invention is the combination of a crosslinked silicone elastomer with spherical particles having a narrow particle size distribution to obtain optical diffusion of lines and wrinkles. The present invention overcomes the failure of the prior art to use such spherical particles alone to reflect light and effectively function as an optical diffuser.

In addition, the present invention may include a secondary component. The secondary component is selected from one or more of the following twelve groups.

1. Retinoids and Rexinoids: Examples of suitable retinoids include retinol, retinoic acid, retinyl palmitate, retinyl propionate, retinyl acetate, isotretinoin as well as synthetic retinoid mimics, and derivatives of the foregoing, as well as others that bind to RAR receptors. Rexinoids include compounds, such as all-trans retinoic acid, 9-cis retinoic acid, phytanic acid and others that bind to RXR receptors.

2. An estrogen synthetase (aromatase) stimulating compound: Examples of such a compound include caffeine and/or derivatives thereof, and any mixture thereof. Caffeine is the more preferred of such compounds.

3. A compound capable of inhibiting 5 alpha-reductase activity: Examples of such a compound include linolenic acid, linoleic acid, finasteride, and mixtures thereof.

4. An exfoliation promoting compound: Suitable examples include alpha hydroxy acids; beta hydroxy acids; oxa acids as disclosed in U.S. Patent No. 5,847,003 (the disclosure of which is incorporated herein by reference); oxa diacids as disclosed in U.S. Patent No. 5,834,513 (the disclosure of which is incorporated herein by reference); mechanical exfoliation compounds, such as bamboo exfoliant extract; salicylic acid; benzoyl peroxide; keto acids, such as pyruvic acid, 2-oxopropanoic acid, 2-oxobutanoic acid, and 2-oxopentanoic acid; and mixtures thereof. The preferred exfoliation promoting compounds are lactic acid, glycolic acid, 3,6,9-trioxaundecanedioic acid, and any mixture thereof. When the present invention includes an exfoliation promoting compound, the composition comprises about 1 wt.% to 20 wt.%, preferably about 1 wt.% to about 15 wt.%, more preferably about 4 wt.% to about 10 wt.%, and most preferably about 4 wt.%, of the exfoliation promoting compound.

5. An ultraviolet (UV) light protecting/sunscreen agent: The sunscreen may be any sunscreen known in the art. Such sunscreens include, but are not limited to, oxybenzone, sulisobenzone, dioxybenzone, menthyl antranilate, para aminobenzoic acid, dea methoxycinnamate, octyl methoxycinnamate, octocrylene, drometrizole trisiloxane, octyl salicylate, homomenthyl salicylate, octyl dimethyl PABA, TEA salicylate, titanium dioxide, zinc oxide, butylmethoxy dibenzoylmethane (avobenzone), methyl benzilidene camphor, octyl triazone, terephthalydiene dicamphor sulfonic acids, ethyl PABA, hydroxy methylphenyl benzotriazole, methylene bis-benzotriazoyltetramethylbutylphenol (MBBT), bis-ethylhexyl oxyphenol methoxyphenol triazine (BEMT), benzophenones, naptholsulphonates, benzoic acid derivatives, salicylates, cinnamic acid derivatives and mixtures thereof. Other sunscreens include those disclosed in U.S. Patent No. 5,000,937, which is incorporated herein. Salts, esters and other derivatives of the aforementioned sunscreen agents, which are compatible with the composition, are also contemplated in practicing the present invention. Co-formulation with an ultraviolet light protecting/sunscreen agent is particularly desirable when the present invention is prepared for consumers who engage in outdoor activities.

6. Barrier function enhancing agents: Examples include ceramides; essential fatty acids and their esters, especially glycerides, α-hydroxy fatty acids and their esters, ω-hydroxy fatty acids and their esters; phospholipids; cholesterol and its esters, such as cholesteryl hemisuccinate, cholesteryl phosphate; and cholestanol and its derivatives. The barrier function enhancing agent can be added to a topical composition either as singular molecular entities or as a complex mixture of lipids derived from either synthetic, animal or plant sources.

7. Collagen enhancing agents: These agents prevent skin sagging by promoting a net increase in collagen, either by reducing collagen breakdown or by promoting collagen formation. Examples of such agents include *Clara* extract (*Sophora augustifolia*), ascorbyl-phoshoryl-cholesterol, ascorbic acid, ascorbic acid derivatives, and mixtures thereof.

8. Elastase inhibitors: Examples of these inhibitors include fatty acids, such as oleic acid, perinaric acid, and Honeysuckle extract (*Lonicera caprifolium*). These inhibitors act to prevent sagging of the skin.

9. Skin lightening agents: Examples include kojic acid, hydroquinone, licorice derivatives, ascorbic acid/ascorbic acid derivatives (e.g. magnesium ascorbyl phosphate), arbutin, bearberry *(Arctostaphylos uva ursi), Glycyrrhiza glabra* and its derivatives, *Chlorella vulgaris* extract, and mixtures thereof.

10. Antioxidants: Examples include compounds having phenolic hydroxy functions, such as ascorbic acid, ascorbic acid derivatives; gallic acid derivatives (e.g. propyl gallate); ferulic acid derivatives (e.g. ethyl ferulate, sodium ferulate); nitrones; N-tertbutyl-nitrone; I-(4-pyridyl-1-oxide)-N-tertbutyl-nitrone; curcumin, tetrahydrocurcumin; 6-hydroxy-2,5,7,tetramethylchroman-2-carboxylic acid; uric acid; reductic acid; tannic acid; rosmarinic acid; tocopherol and its derivatives; catechins; and mixtures thereof. Other suitable antioxidants are those that have one or more thiol functions (-SH), in either reduced or non-reduced form, such as glutathione, lipoic acid, thioglycolic acid, and other sulfhydryl compounds. The antioxidant may be inorganic, such as sulfites, bisulfites, metabisulfite, or other inorganic salts and acids containing sulfur.

11. Skin cooling agents: Examples include vanillyl butylamid, capsaicin and mixtures thereof.

12. Phytoestrogen: Described in PCT WO 00/13661, which is incorporated herein by reference.

The addition of the secondary component enhances the dermatological benefits achieved by and the utilization of compositions of the present invention. The secondary component must be present in an amount effective to provide its intended function. The compositions of the present invention preferably include at least two secondary components, with each secondary component being selected from a different group.

The present compositions may include a vehicle. The vehicle is in the form of a solid, solution, essence, serum, pencil, spray, lotion, emulsion, cream, micro-emulsion, gel, ointment, patch, stick and tape.

The compositions of the present invention can include other cosmetic and pharmaceutical actives and excipients. Such suitable cosmetic and pharmaceutical agents include, but are not limited to, one or more erythromycins, tetracyclines, salicylic acids, antifungals, vitamins, antiinflammatory agents, antimicrobials, analgesics, nitric oxide synthase inhibitors, insect repellents, self-tanning agents, surfactants, moisturizers, stabilizers, preservatives, antiseptics, chelating agents, thickeners, emulsifiers, lubricants, humectants, chelating agents, skin penetration enhancers, skin cooling agents, emollients, fragrances and colorants.

Various modifications and alterations to the present invention may be appreciated based on a review of this application. These changes and additions are intended to be within the scope and the spirit of the present invention as defined by the following claims.

## Claims

1. A cosmetic composition comprising:
a crosslinked silicone elastomer;
a plurality of substantially spherical particles having a particle size distribution of about 24 microns; and
a vehicle.

2. The cosmetic composition of claim 1, wherein the substantially spherical particles are spherical.

3. The cosmetic composition of claim 1, wherein the particle size distribution is about 15 microns.

4. The cosmetic composition of claim 1, wherein the particle size distribution is about 7 microns.

5. The cosmetic composition of claim 1, wherein the particle size distribution is about 2 microns.

6. The cosmetic composition of claim 1, wherein the substantially spherical particles are uniform in diameter.

7. The cosmetic composition of claim 1, wherein the particle size range is about 1 micron to about 25 microns.

8. The cosmetic composition of claim 1, wherein the particle size range is about 5 microns to about 20 microns.

9. The cosmetic composition of claim 1, wherein the particle size range is about 8 microns to about 15 microns.

10. The cosmetic composition of claim 1, wherein the particle size range is about 8 microns to about 10 microns.

11. The cosmetic composition of claim 1, wherein the crosslinked silicone elastomer is present in an amount from about 0.01 wt.% to about 10 wt.% of the total weight of the composition.

12. The cosmetic composition of claim 1, wherein the substantially spherical particles are present in an amount from about 0.01 wt.% to about 10 wt.% of the total weight of the composition.

13. The cosmetic composition of claim 1, wherein the substantially spherical particles are present in an amount from about 0.5 wt.% to about 5 wt.% of the total weight of the composition.

14. The cosmetic composition of claim 1, wherein the substantially spherical particles are selected from the group consisting of silica, nylon, boron nitride, teflon, polyurethane powder, silicone powder, talc, mica, serecite, and mixtures thereof

15. The cosmetic composition of claim 1, wherein the crosslinked silicone elastomer is selected from the group consisting of: dimethicone crosspolymer; organopolysiloxane; polysilicone-11; and dimethicone/vinyl dimethicone crosspolymer; and mixtures thereof.

16. The cosmetic composition of claim 1, further comprising a secondary component selected from the group consisting of:
(i) an estrogen synthetase stimulating compound;
(ii) a 5 alpha-reductase activity inhibiting compound;
(iii) an exfoliation-promoting compound;
(iv) an ultraviolet (UV) light protecting/sunscreen agent;
(v) a retinoid;
(vi) a hirsutism inhibiting agent;
(vii) a barrier function enhancing agent;
(viii) a collagen enhancing agent;
(ix) an elastase inhibitor;
(x) a skin lightening agent
(xi) an antioxidant;
(xii) a skin cooling agent;
(xiii) a phytoestrogen; and
(xiv) mixtures thereof.

17. The cosmetic composition of claim 1, wherein the vehicle is selected from the group consisting of a solid, solution, essence, serum, pencil, spray, lotion, emulsion, cream, micro-emulsion, gel, ointment, patch, stick and tape.

18. A method of improving the aesthetic appearance of skin comprising topically applying the cosmetic composition as in claim 1.

19. The method of claim 18, wherein the improvement in aesthetic appearance includes at least one of the following:
a. improving the appearance of skin texture;
b. decreasing the appearance of fine lines and wrinkles;
c. improving skin tone;
d. decreasing the appearance of pore size;
e. minimizing the appearance of skin discoloration;
f. restoring skin luster; and
g. minimizing signs of fatigue.

20. The method of improving the aesthetic appearance of skin comprising topically applying the cosmetic composition as in claim 16.
